Europäisches Patentamt
European Patent Office
Office européen des brevets

(19) (11) Publication number: **0 247 464 A1**

(12) # EUROPEAN PATENT APPLICATION

(21) Application number: **87107108.0**

(22) Date of filing: **16.05.87**

(51) Int. Cl.[4]: **C 07 D 215/56**, A 61 K 31/47 // C07C69/738, C07C119/06

(30) Priority: **19.05.86 GB 8612137**

(43) Date of publication of application: **02.12.87 Bulletin 87/49**

(84) Designated Contracting States: **AT BE CH DE ES FR GB GR IT LI LU NL SE**

(71) Applicant: **Fujisawa Pharmaceutical Co., Ltd., 3, Doshomachi 4-chome Higashi-ku, Osaka-shi, Osaka 541 (JP)**

(72) Inventor: **Teraji, Tsutomu, 20-6, Kofudai 6-chome Toyono-cho, Toyono-gun Osaka (JP)**
Inventor: **Matsushima, Hiroshi, 9-15-302, Nishishoji 2-chome, Minoo-shi Osaka (JP)**
Inventor: **Yamamura, Atsushi, 12-27-301, Higashimikuni 2-chome, Yodogawa-ku Osaka (JP)**

(74) Representative: **Türk, Gille, Hrabal, Bruckner Strasse 20, D-4000 Düsseldorf 13 (DE)**

(54) **New quinolone compounds and a process for the preparation thereof.**

(57) Compounds of the formula:

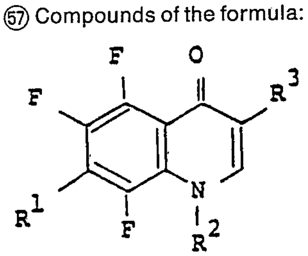

(I)

wherein

$R^1$ is di(lower)alkylamino, piperazinyl, piperazinyl having lower alkyl, morpholinyl, pyrrolidinyl or piperidyl,

$R^2$ is cyclo(lower)alkyl or phenyl having lower alkyl and hydroxy, and

$R^3$ is carboxy or protected carboxy, and physiologically acceptable salts thereof, processes for the preparation thereof, pharmaceutical compositions containing them and their use for treatment of infectious diseases.

EP 0 247 464 A1

# NEW QUINOLONE COMPOUNDS AND A PROCESS FOR THE PREPARATION THEREOF

The present invention relates to new quinolone compounds and physiologically acceptable salts thereof. More particularly, it relates to new quinolone compounds and physiologically acceptable salts thereof, which have antimicrobial activities and to a process for the preparation thereof, to antimicrobial composition comprising the same, and to a method of using the same therapeutically in the treatment of infectious diseases in human being or animals, especially in animals.

Accordingly, it is one object of the present invention to provide new quinolone compounds and physiologically acceptable salts thereof, which are active against a number of pathogenic microorganisms.

Another object of the present invention is to provide a process for the preparation of new quinolone compounds and salts thereof.

A further object of the present invention is to provide an antimicrobial composition comprising,

as active ingredients, said new quinolone compounds and physiologically acceptable salts thereof.

Still further object of the present invention is to provide a method for the treatment of infectious diseases caused by pathogenic microorganisms in human being or animals, especially in animals.

The object quinolone compounds of the present invention are novel and can be represented by the following general formula (I).

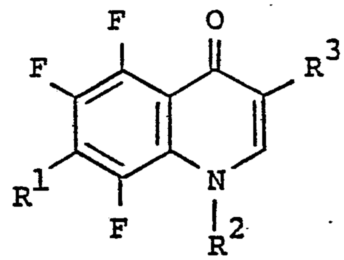

(I)

wherein $R^1$ is di(lower)alkylamino, piperazinyl, piperazinyl having lower alkyl, morpholinyl, pyrrolidinyl or piperidyl,

$R^2$ is cyclo(lower)alkyl or phenyl having lower alkyl and hydroxy, and

$R^3$ is carboxy or protected carboxy.

According to the present invention, the new quinolone compounds (I) can be prepared by the processes which are illustrated in the following reaction schemes.

<u>Process 1</u> :

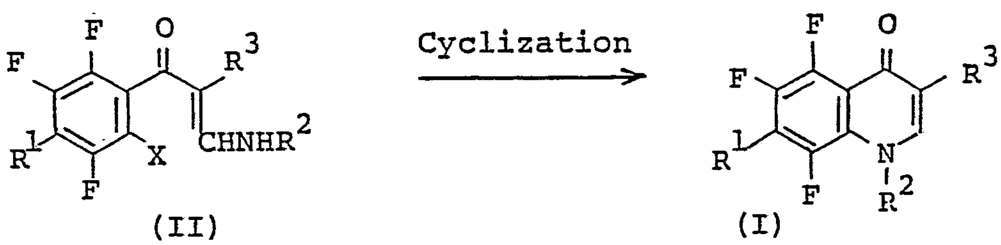


(II) or salts thereof

(I) or salts thereof

Process 2 :

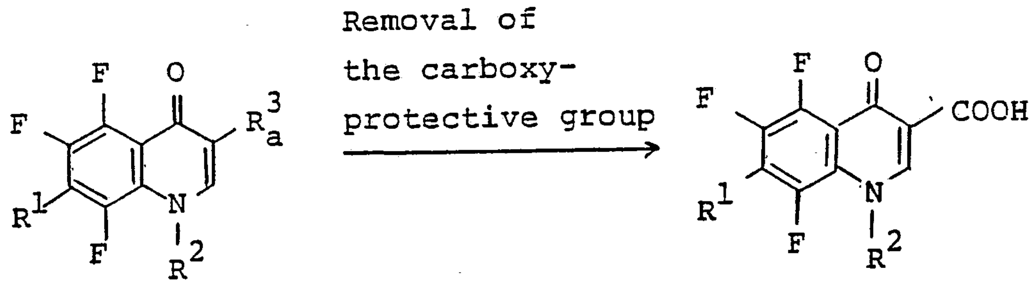


(I-a) or salts thereof

(I-b) or salts thereof

Process 3 :

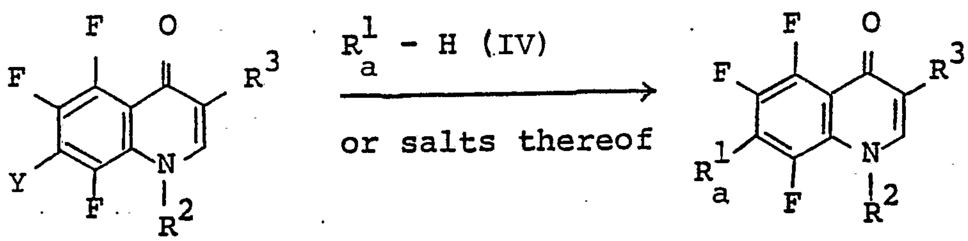


(III) or salts thereof

(I-c) or salts thereof

wherein $R^1$, $R^2$ and $R^3$ are each as defined above,

X and Y are each halogen,

$R^1_a$ is di(lower)alkylamino, 1-piperazinyl, 1-piperazinyl having lower alkyl, morpholino, 1-pyrrolidinyl or piperidino, and

$R^3_a$ is protected carboxy.

The starting compound (II) or salts thereof can bepreparaed according to the methods disclosed in the following Preparations or the similar manners thereto.

Suitable physiologically acceptable salts of the object compounds (I) are conventional ones and include a metal salt such as an alkali metal salt (e.g. sodium salt, potassium salt, etc.) and an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N'-dibenzylethylenediamine salt, etc.), an organic acid salt (e.g. acetate, trifluoroacetate, maleate, tartrate, methanesulfonate, benzenesulfonate, formate, toluenesulfonate, etc.), an inorganic acid salt (e.g. hydrochloride, hydrobromide, hydriodide, sulfate, phosphate, etc.), a salt with an amino acid (e.g. arginine, aspartic acid, glutamic acid, etc.), and the like.

In the above descriptions of the present specification, suitable examples and illustrations of the various definitions which the present invention include within scope thereof are explained in detail as follows.

The term "lower" is intended to mean 1 to 6 carbon atom(s) unless otherwise indicated.

Suitable "halogen" may include chlorine, bromine, fluorine and iodine, in which the preferred one may be fluorine.

Suitable "di(lower)alkylamino" may include dimethylamino, diethylamino, dipropylamino, diisopropylamino, dibutylamino, dipentylamino, dihexylamino, ethylmethylamino, and the like, in which the preferred one may be di$(C_1-C_4)$alkylamino.

Suitable "piperazinyl" may include 1-piperazinyl and the like.

Suitable "piperazinyl having lower alkyl" may include lower alkyl-1-piperazinyl (e.g. 4-methyl-1-piperazinyl, 4-ethyl-1-piperazinyl, 4-propyl-1-piperazinyl, 4-isopropyl-1-piperazinyl, 4-hexyl-1-piperazinyl, 3-methyl-1-piperazinyl, 3-ethyl-1-piperazinyl, 2-ethyl-1-piperazinyl, etc.), and the like, in which the preferred one may be 4-(lower)-alkyl-1-piperazinyl and 3-(lower)alkyl-1-piperazinyl, and the more preferred one may be 4-($C_1$-$C_4$)alkyl-1-piperazinyl and 3-($C_1$-$C_4$)alkyl-1-piperazinyl.

Suitable "morpholinyl" may include morpholino, and the like.

(to be continued to the next page)

Suitable "pyrrolidinyl" may include 1-pyrrolidinyl, and the like.

Suitable "piperidyl" may include piperidino, and the like.

Suitable "1-piperazinyl having lower alkyl" may include lower alkyl-1-piperazinyl as exemplified above.

Suitable "cyclo(lower)alkyl" may include the one having 3 to 6 carbon atoms such as cyclopropyl, cyclobutyl, cyclopentyl and cyclohexyl, in which the preferred one may be cyclopropyl.

Suitable "phenyl having lower alkyl and hydroxy" may include 2-methyl-4-hydroxyphenyl, 2-ethyl-4-hydroxyphenyl, 2-propyl-4-hydroxyphenyl, 2-isopropyl-4-hydroxyphenyl, 2-butyl-4-hydroxyphenyl, 2-hexyl-4-hydroxyphenyl, 2-methyl-3-hydroxyphenyl, 3-methyl-4-hydroxyphenyl, 2-methyl-6-hydroxyphenyl, and the like, in which the preferred one may be 2-(lower)alkyl-4-hydroxyphenyl and the more preferred one may be 2-($C_1$-$C_4$)-alkyl-4-hydroxyphenyl.

Suitable "protected carboxy" may include esterified carboxy in which said ester may be the ones such as:

lower alkyl ester (e.g. methyl ester, ethyl ester, propyl ester, isopropyl ester, butyl ester, isobutyl ester, t-butyl ester, pentyl ester, t-pentyl ester, hexyl ester, etc.);

lower alkenyl ester (e.g. vinyl ester, allyl ester, etc.);

lower alkynyl ester (e.g. ethynyl ester, propynyl ester, etc.);

mono(or di or tri)-halo(lower)alkyl ester (e.g. 2-iodoethyl ester, 2,2,2-trichloroethyl ester, etc.);

lower alkanoyloxy(lower)alkyl ester (e.g. acetoxymethyl ester, propionyloxymethyl ester, 1-acetoxypropyl ester, valeryloxymethyl ester, pivaloyloxymethyl ester, hexanoyloxymethyl ester, 1-acetoxyethyl ester, 2-propionyloxyethyl ester, 1-isobutyryloxyethyl ester, etc.);

lower alkanesulfonyl(lower)alkyl ester (e.g. mesylmethyl ester, 2-mesylethyl ester, etc.);

ar(lower)alkyl ester, for example, phenyl(lower)-alkyl ester which may be substituted with one or more suitable substituent(s) [e.g. benzyl ester, 4-methoxybenzyl ester, 4-nitrobenzyl ester, phenethyl ester, trityl ester, diphenylmethyl ester, bis(methoxyphenyl)-methyl ester, 3,4-dimethoxybenzyl ester, 4-hydroxy-3,5-ditertiarybutylbenzyl ester, etc.];

lower alkoxycarbonyloxy(lower)alkyl ester (e.g. methoxycarbonyloxymethyl ester, ethoxycarbonyloxymethyl ester, ethoxycarbonyloxyethyl ester, etc.) which may be substituted with azido;

a heterocyclic ester, preferably benzotetrahydrofuryl ester which may be substituted with oxo group, more preferably phthalidyl ester;

aroyloxy(lower)alkyl ester (e.g. benzoyloxymethyl ester, benzoyloxyethyl ester, toluoyloxyethyl ester, etc.);

aryl ester which may have one or more suitable substituent(s) (e.g. phenyl ester, tolyl ester, tert-butylphenyl ester, xylyl ester, mesityl ester, cumenyl ester, etc.), and the like.

The preferred example of the protected carboxy thus defined may be lower alkoxycarbonyl and the more preferred one may be $C_1$-$C_4$ alkoxycarbonyl.

The process for preparing the object compounds (I) of the present invention is explained in detail in the following.

Process 1 :

The compounds (I) or salts thereof can be prepared by subjecting the compound (II) or salts thereof to cyclization reaction.

The starting compound (II) used in this process is new and can be prepared by the methods explained in the

Preparations as mentioned later or a conventional manner thereof.

Suitable salts of the compound (II) may include the same as those exemplified for the compounds (I).

The cyclization reaction can be carried out in the presence of a base, for example, an inorganic base such as alkali metal hydroxide (e.g. sodium hydroxide, potassium hydroxide, etc.), alkaline earth metal hydroxide (e.g. magnesium hydroxide, calcium hydroxide, etc.), alkali metal carbonate (e.g. sodium carbonate, potassium carbonate, etc.), alkaline earth metal carbonate (e.g. magnesium carbonate, calcium carbonate, etc.), alkali metal bicarbonate (e.g. sodium bicarbonate, potassium bicarbonate, etc.), alkali metal acetate (e.g. sodium acetate, potassium acetate, etc.), alkaline earth metal phosphate (e.g. magnesium phosphate, calcium phosphate, etc.), alkali metal hydrogen phosphate (e.g. disodium hydrogen phosphate, dipotassium hydrogen phosphate, etc.), or the like, and an organic base such as trialkylamine (e.g. trimethylamine, triethylamine, etc.), picoline, N-methylpyrrolidine, or the like.

This reaction is usually carried out in a solvent which does not adversely affect the reaction such as N,N-dimethylformamide, under cooling to heating, preferably under ice-cooling to warming.

Process 2 :

The compound (I-b) or salts thereof can be prepared by removing the carboxy-protective group from the compound (I-a) or salts thereof.

Suitable salts of the compounds (I-a) and (I-b) may include the same as those exemplified for the compounds (I).

The present reaction is carried out in a conventional method such as hydrolysis, and the like.

Hydrolysis is preferably carried out in the presence

of a base or an acid. Suitable base may include an alkali metal hydroxide (e.g. sodium hydroxide, potassium hydroxide, etc.), an alkaline earth metal hydroxide (e.g. magnesium hydroxide, calcium hydroxide, etc.), an alkali metal carbonate (e.g. sodium carbonate, potassium carbonate, etc.), an alkaline earth metal carbonate (e.g. magnesium carbonate, calcium carbonate, etc.), an alkali metal bicarbonate (e.g. sodium bicarbonate, potassium bicarbonate, etc.), and the like.

Suitable acid may include an organic acid (e.g. formic acid, acetic acid, propionic acid, trifluoroacetic acid, benzenesulfonic acid, p-toluenesulfonic acid, etc.) and an inorganic acid (e.g. hydrochloric acid, hydrobromic acid, sulfuric acid, phosphoric acid, etc.). The acidic hydrolysis using trifluoroacetic acid is usually accelerated by addition of cation trapping agent (e.g. phenol, anisole, etc.).

This reaction is usually carried out in a solvent which does not adversely affect the reaction such as acetic acid, under cooling to heating, preferably under warming to refluxing in the solvent.

Process 3 :

The compound (I-c) or salts thereof can be prepared by reacting the compound (III) or salts thereof with the compound (IV) or salts thereof.

Suitable salts of the compounds (I-c) and (III) may include the same as those exemplified for the compound (I), and those of the compounds (IV) may include the organic or inorganic acid salts for the compounds (I).

This reaction may be carried out in the presence of a base such as those exemplified in the explanation of Process 1.

This reaction may be usually carried out in a solvent which does not adversely affect the reaction such as water, methanol, ethanol, propanol, N,N-dimethylformamide, dimethylsulfoxide or a mixture thereof, under cooling to heating, preferably at ambient temperature to under refluxing in the solvent.

The object compounds (I) and salts thereof obtained according to the processes 1 to 3 as explained above can be isolated and purified in a conventional manner, for example, extraction, precipitation, fractional crystallization, recrystallization, chromatography, and the like.

The object compounds (I) and physiologically acceptable salts thereof of the present invention are novel compounds which exhibit high antimicrobial activity and inhibit the growth of a wide variety of pathogenic microorganisms including Gram-positive and Gram-negative bacteria and mycoplasma in human being or animals, especially in animals, for example, economic domestic animals such as poultry (e.g. chicken, turkey, duck, quail, etc.), cattle, pig, sheep, goat, rabbit, mink, dog, cat, mouse, rat, fish, and the like. Therefore, the compounds (I) and physiologically acceptable salts thereof are useful as antimicrobial agents.

Among the object compounds (I), the compound (I-d) of the formula :

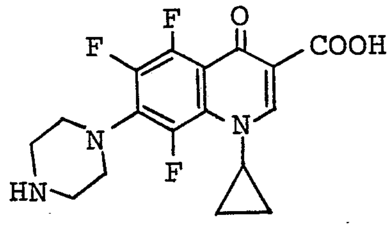

(I-d)

and physiologically acceptable salts thereof exhibit especially potent antimicrobial activity and, further, the toxicity thereof is extremely low.

Accordingly, the compound (I-d) is a particularly good antimicrobial agent for human being and animals, especially for animals.

In order to illustrate the usefulness of the object compounds (I), the test data on the antimicrobial activity and the acute toxicity of the representative compound of the present invention are shown below.

Test 1: Minimal Inhibitory Concentration (MIC)

(A) Test Methods

(to be continued to the next page)

(a) Antimycoplasmal activity

MICs of test compound were obtained by the agar dilution method.

Agar plates contained two-fold decremental dilutions of the test compound, ranging from 0.05 to 100 μg/ml. The inoculum (0.01 ml, containing approximately $10^5$ CFU/ml of liquid medium which had been cultured at 37°C for 3 days) was plated on moisture-free media surfaces. Plates were placed in a humidified atmosphere of 5 per cent carbon dioxide and 95 per cent air, and incubated at 37°C for 3 to 4 days. The colonies grown on the plate were observed under a low microscopic magnification (x60). The lowest concentration of a drug that completely inhibited the growth of colonies was regarded as the MIC of the drug.

(b) Antibacterial activity

MICs against *Vibrio anguillarum* originated in fish diseases were obtained by the following method.

An overnight culture broth of each strain was diluted 10-fold (approximately $10^6$ cells/ml) and was stamped on 2.5% sodium chloride added Heart Infusion Agar containing graded concentrations of the test compounds, and MICs were expressed in terms of μg/ml after 20 hours incubation at 25°C.

(B) Test Compound

1-Cyclopropyl-5,6,8-trifluoro-7-(1-piperazinyl)-4-quinolone-3-carboxylic acid [aforesaid compound (I-d)]

(C) Test Results

| Test Methods | Test Microorganisms | MIC (μg/ml) |
|---|---|---|
| Antimycoplasmal activity | Mycoplasma pulmonis PG-22 | 0.10 |
| Antibacterial activity | Vibrio anguillarum NP-1 | 0.10 |

Test 2: Acute Toxicity

(A) Test Method

The test compound (16 mg) was dissolved in 1N NaOH (0.1 ml) and steril distilled water (3.9 ml).

Said test compound was injected into lateral vein (dosage: 100 mg/kg) to each ICR-strain male mouse (one group - 5 mice; weight - 20 to 25 g) and each mouse was observed for 7 days.

(B) Test Compound

1-Cyclopropyl-5,6,8-trifluoro-7-(1-piperazinyl)-4-quinolone-3-carboxylic acid [aforesaid compound (I-d)]

(C) Test Results

| Survivals / test mice |
|---|
| 5 / 5 |

(to be continued to the next page)

The object compounds (I) and physiologically acceptable salts thereof of the present invention can be administered to human being or animals in a conventional manner.

Especially, with respect to the administration to aminals, the compounds (I) and physiologically acceptable salts thereof are preferably administered orally to animals, and the compounds (I) and physiologically acceptable salts thereof may be generally administered as they are or in admixture with a suitable carrier (e.g. water, etc.) or in admixture with an animal nutrition source, i.e. feed. More particularly, the compounds (I) and physiologically acceptable salts thereof may be administered to as a drinking water in the form of aqueous solution, or as a ration in the form of the composition which comprises the compounds (I) and physiologically acceptable salts thereof and animal feed and sometimes the other feed additive.

In connection with the form of administering the animal feed composition as mentioned above, the ration comprising the compounds (I) and physiologically acceptable salts thereof can be prepared in a conventional manner; namely, by admixing the compounds (I) and physiologically acceptable salts thereof with basal ration.. And, as the basal ration, natural feed and

(to be continued to the next page)

assorted feed can be used, including dry feeds, liquid feed, pelleted feed, or the like. As preferred basal ration, there is preferably used the assorted feed which comprises one or more conventional feeds such as corn, rice, wheat, milo, soybean meal, cottonseed meal, wheat bran, defatted rice bran, fish meal, skim milk, dried whey, oils, fats, alfalfa meal or the like and one or more of the conventional feed additives such as tricalcium carbonate, sodium chloride, choline chloride, vitamin (e.g. vitamin A, vitamin D, vitamin E, vitamin $B_1$, vitamin $B_2$, vitamin $B_6$, vitamin $B_{12}$, calcium pantothenate, nicotinamide, folic acid, etc.), amino acid (e.g. lysine, methionine, etc.), mineral source (e.g. magnesium sulfate, ferrous sulfate, copper sulfate, zinc sulfate, potassium iodide, cobalt sulfate, etc.), or the like.

The amounts of the compounds (I) and physiologically acceptable salts thereof in the basal rations which are fed to the animals may be varied over a very wide range depending upon the kind, growth period, etc. of the animals and the severity of infection of animals, or the like. Preferred levels are in an amount between about 1 p.p.m. and about 5000 p.p.m., more preferably between about 10 p.p.m. and about 1000 p.p.m.

The new quinolone compounds (I) of this invention may also be administered non-orally to animals, by the methods such as intravenous, intramuscular, intraperitoneal or subcutaneous injection, spray, or the like. In such a non-oral administration, the compounds (I) and physiologically acceptable salts thereof may be preferably administered in admixture with a suitable carrier such as water, or the like.

Dosage of the compounds (I) and physiologically acceptable salts thereof to animals is varied depending

upon the kind, the severity of infection, etc. of the animals, and its preferred dosage may usually be selected from the range of about 0.1-1000 mg/kg/day as the amount of the compounds (I) and physiologically acceptable salt thereof.

The following examples are given for the purpose of illustrating the present invention in detail.

Preparation 1

A solution of ethyl 3-(2,3,4,5,6-pentafluorophenyl)-3-oxopropionate (16.0 g) in triethyl orthoformate (12.6 g) and acetic anhydride (15.1 g) was refluxed for 2 hours. The reaction mixture was evaporated under reduced pressure to give ethyl 2-ethoxymethylene-3-(2,3,4,5,6-pentafluorophenyl)-3-oxopropionate (18.0 g).

IR (film) : 1705, 1673 $cm^{-1}$

Preparation 2

Ethyl 2-ethoxymethylene-3-(2,3,4,5,6-pentafluorophenyl)-3-oxopropionate (1.0 g) was dissolved in ethanol (20 ml). Cyclopropylamine (340 mg) was added to the solution, followed by stirring for 2 hours. The precipitate was collected by filtration and dried to give ethyl 2-cyclopropylaminomethylene-3-(2,3,4,5,6-pentafluorophenyl)-3-oxopropionate (1.1 g).

IR (Nujol) : 3190, 1693, 1630 $cm^{-1}$

NMR ($CDCl_3$) δppm : 11.4-10.7 (bs, 1H), 8.43 (s, 0.6H), 8.20 (s, 0.4H), 4.05 (q, 2H, J=7Hz), 3.3-2.7 (m, 1H), 1.11 (t, 3H, J=7Hz), 1.2-0.8 (m, 4H)

Preparation 3

Ethyl 3-(2,3,4,5,6-pentafluorophenyl)-2-(4-hydroxy-2-methylanilinomethylene)-3-oxopropionate (5.8 g) was

obtained by reacting ethyl 2-ethoxymethylene-3-(2,3,4,5,6-pentafluorophenyl)-3-oxopropionate (7.0 g) with 4-hydroxy-2-methylaniline (3.8 g) according to a similar manner to that of Preparation 2.

mp : 149 to 151°C

IR (Nujol) : 3270, 1670, 1625 $cm^{-1}$

NMR ($CDCl_3$) δppm : 8.70 (s, 0.6H), 8.50 (s, 0.4H), 7.36-7.06 (m, 1H), 6.90-6.60 (m, 2H), 4.60 (q, 2H, J=7Hz), 2.37 (s, 3H), 1.17 (t, 3H, J=7Hz)

Preparation 4

Ethyl 2-cyclopropylaminomethylene-3-(2,3,4,5,6-pentafluorophenyl)-3-oxopropionate (1.0 g) was dissolved in N,N-dimethylformamide (12 ml), and thereto was added potassium carbonate (0.47 g) under ice-cooling. After the mixture was stirred at ambient temperature for 2 hours, the reaction mixture was poured into ice-water. The precipitated crystals were collected by filtration, washed with water and then dried to obtain ethyl 1-cyclopropyl-5,6,7,8-tetrafluoro-4-quinolone-3-carboxylate (0.94 g).

mp : 168 to 169°C

IR (Nujol) : 1730, 1632 $cm^{-1}$

NMR ($CDCl_3$) δppm : 8.46 (s, 1H), 4.33 (q, 2H, J=7Hz), 4.03-3.70 (m, 1H), 1.38 (t, 3H, J=7Hz), 1.35-1.10 (m, 4H)

Preparation 5

Ethyl 5,6,7,8-tetrafluoro-1-(4-hydroxy-2-methylphenyl)-4-quinolone-3-carboxylate (1.59 g) was obtained by subjecting ethyl 3-(2,3,4,5,6-pentafluorophenyl)-2-(4-hydroxy-2-methylanilinomethylene)-3-oxopropionate (1.7 g) to cyclization reaction in the presence of potassium carbonate (0.57 g) according to a

similar manner to that of Preparation 5.

IR (Nujol) : 3300, 1695, 1630 $cm^{-1}$

Preparation 6

Ethyl 1-cyclopropyl-5,6,7,8-tetrafluoro-4-quinolone-3-carboxylate (0.94 g) was added to a mixture of glacial acetic acid (7 ml) and conc. hydrochloric acid (2 ml), followed by refluxing for 2 hours. After cooling, the reaction mixture was poured into ice-water (20 ml), and the precipitated crystals were collected by filtration, washed with water and then dried to obtain 1-cyclopropyl-5,6,7,8-tetrafluoro-4-quinolone-3-carboxylic acid (730 mg).

mp : 159 to 163°C

IR (Nujol) : 1726, 1617 $cm^{-1}$

NMR ($DMSO-d_6$) δppm : 14.0 (bs, 1H), 8.66 (s, 1H), 4.3-3.8 (m, 1H), 1.20 (m, 4H)

Preparation 7

5,6,7,8-Tetrafluoro-1-(4-hydroxy-2-methylphenyl)-4-quinolone-3-carboxylic acid (0.75 g) was obtained by reacting ethyl 5,6,7,8-tetrafluoro-1-(4-hydroxy-2-methylphenyl)-4-quinolone-3-carboxylate (1.2 g) with conc. hydrochloric acid (3 ml) according to a similar manner to that of Preparation 6.

mp : 280 to 285°C

IR (Nujol) : 1700, 1620 $cm^{-1}$

Example 1

To a solution of 1-cyclopropyl-5,6,7,8-tetrafluoro-4-quinolone-3-carboxylic acid (300 mg) in water (6 ml) was added piperazine (257 mg) at ambient temperature, and the mixture was stirred at 60°C for 3 hours. The reaction mixture was cooled to 30°C and adjusted to pH 9

with diluted hydrochloric acid. The precipitated crystals were collected by filtration, washed with water and then dried to give 1-cyclopropyl-5,6,8-trifluoro-7-(1-piperazinyl)-4-quinolone-3-carboxylic acid (120 mg).

mp : >300°C

IR (Nujol) : 3350, 1730, 1630 $cm^{-1}$

NMR ($CF_3COOH$) δ ppm : 1.2-1.9 (4H, m), 3.8 (4H, brs) 4.1 (4H, brs), 4.3-4.8 (1H, m), 7.3-8.3 (2H, brs), 9.4 (1H, s)

(to be continued to the next page)

Example 2

To a suspension of ethyl 1-cyclopropyl-5,6,7,8-tetrafluoro-4-quinolone-3-carboxylate (10 g) in dimethyl sulfoxide (30 ml) was added piperazine (5.22 g) at ambient temperature, and the mixture was stirred at 40 to 45°C for half an hour. To the reaction mixture was added water (60 ml) dropwise below 25°C and the mixture was stirred for 30 minutes in an ice bath. The resulting precipitates were filtered, dissolved in 1% aqueous hydrochloric acid (130 ml) and washed with methylene chloride. The aqueous solution was cooled to 10°C and adjusted to pH 11 with 24% aqueous sodium hydroxide. The precipitated crystals were filtered, washed with water and dried to give ethyl 1-cyclopropyl-5,6,8-trifluoro-7-(1-piperazinyl)-4-quinolone-3-carboxylate (9.8 g).

mp : 207-210°C

IR (Nujol) : 3170, 1722, 1625 $cm^{-1}$

NMR ($CDCl_3$) δ ppm : 1.0-1.4 (4H, m), 1.37 (3H, t, J=7.2Hz), 2.8-3.2 (4H, m), 3.2-3.5 (4H, m), 3.6-4.1 (1H, m), 4.33 (2H, q, J=7.2Hz), 8.42 (1H, s)

Example 3

To a suspension of ethyl 1-cyclopropyl-5,6,8-trifluoro-7-(1-piperazinyl)-4-quinolone-3-carboxylate (1.5 g) in 60% aqueous methanol (12.5 ml) was added a solution of sodium hydroxide (0.3 g) in water (2.5 ml). The mixture was stirred at 50 to 55°C for one hour and adjusted to pH 7 with 10% aqueous hydrochloric acid under refluxing and stirring. The reaction mixture was cooled to 10°C and the precipitated crystals were collected by filtration and dried to give 1-cyclopropyl-5,6,8-trifluoro-7-(1-piperazinyl)-4-quinolone-3-carboxylic acid (1.26 g).

mp : >300°C

IR (Nujol) : 3350, 1730, 1630 $cm^{-1}$

NMR ($CF_3COOH$) δ ppm : 1.2-1.9 (4H, m), 3.8 (4H, brs), 4.1 (4H, brs), 4.3-4.8 (1H, m), 7.3-8.3 (2H, brs), 9.4 (1H, s)

Example 4

2-Cyclopropylaminomethylene-3-[2,3,5,6-tetrafluoro-4-(1-piperazinyl)phenyl]-3-oxopropionic acid (1.5 g) was dissolved in N,N-dimethylformamide (20 ml), and thereto was added potassium carbonate (1.0 g) under ice-cooling. After the mixture was stirred at ambient temperature for 2 hours, the reaction mixture was poured into ice-water and adjusted to pH 7 with 10% hydrochloric acid solution. The precipitated crystals were collected by filtration, washed with water and then dried to obtain 1-cyclopropyl-5,6,8-trifluoro-7-(1-piperazinyl)-4-quinolone-3-carboxylic acid (0.1 g).

mp : >300°C

IR (Nujol) : 3350, 1730, 1630 $cm^{-1}$

NMR ($CF_3COOH$) $\delta$ ppm : 1.2-1.9 (4H, m), 3.8 (4H, brs), 4.1 (4H, brs), 4.3-4.8 (1H, m), 7.3-8.3 (2H, brs), 9.4 (1H, s)

Example 5

1-Cyclopropyl-5,6,8-trifluoro-7-(4-methyl-1-piperazinyl)-4-quinolone-3-carboxylic acid (280 mg) was obtained by reacting 1-cyclopropyl-5,6,7,8-tetrafluoro-4-quinolone-3-carboxylic acid (300 mg) with N-methylpiperazine (199 mg) according to a similar manner to that of Example 1.

mp : 248 to 250°C

IR (Nujol) : 1732, 1635 $cm^{-1}$

Example 6

To a solution of 1-cyclopropyl-5,6,7,8-tetrafluoro-4-quinolone-3-carboxylic acid (300 mg) in dimethylsulfoxide (5 ml) was added morpholine (174 mg) at ambient temperature, and the mixture was stirred at 40 to 45°C for an hour. To the reaction mixture was added ice-water (30 ml), and the resultant solution

was neutralized with diluted hydrochloric acid. The precipitated crystals were collected by filtration, washed with water and then dried to obtain 1-cyclopropyl-5,6,8-trifluoro-7-morpholino-4-quinolone-3-carboxylic acid (320 mg).

mp : >300°C

IR (Nujol) : 1727, 1630 $cm^{-1}$

Example 7

1-Cyclopropyl-5,6,8-trifluoro-7-(1-pyrrolidinyl)-4-quinolone-3-carboxylic acid (290 mg) was obtained by reacting 1-cyclopropyl-5,6,7,8-tetrafluoro-4-quinolone-3-carboxylic acid (300 mg) with pyrrolidine (142 mg) according to a similar manner to that of Example 6.

mp : >300°C

IR (Nujol) : 1725, 1635 $cm^{-1}$

(to be continued to the next page)

Example 8

5,6,8-Trifluoro-1-(4-hydroxy-2-methylphenyl)-7-(1-piperazinyl)-4-quinolone-3-carboxylic acid (130 mg) was obtained by reacting 5,6,7,8-tetrafluoro-1-(4-hydroxy-2-methylphenyl)-4-quinolone-3-carboxylic acid (200 mg) with piperazine (187 mg) according to a similar manner to that of Example 6.

mp : >300°C

IR (Nujol) : 3450, 1640 $cm^{-1}$

Example 9

5,6,8-Trifluoro-1-(4-hydroxy-2-methylphenyl)-7-(4-methyl-1-piperazinyl)-4-quinolone-3-carboxylic acid (480 mg) was obtained by reacting 5,6,7,8-tetrafluoro-1-(4-hydroxy-2-methylphenyl)-4-quinolone-3-carboxylic acid (500 mg) with N-methyl-piperazine (272 mg) according to a similar manner to that of Example 6.

mp : >300°C

IR (Nujol) : 1730, 1635 $cm^{-1}$

Example 10

5,6,8-Trifluoro-1-(4-hydroxy-2-methylphenyl)-7-(3-methyl-1-piperazinyl)-4-quinolone-3-carboxylic acid (450 mg) was obtained by reacting 5,6,7,8-tetrafluoro-1-(4-hydroxy-2-methylphenyl)-4-quinolone-3-carboxylic acid (500 mg) with 2-methylpiperazine (267 mg) according to a similar manner to that of Example 6.

mp : >300°C

IR (Nujol) : 1627 $cm^{-1}$

Example 11

5,6,8-Trifluoro-1-(4-hydroxy-2-methylphenyl)-7-morpholino-4-quinolone-3-carboxylic acid (550 mg) was obtained by reacting 5,6,7,8-tetrafluoro-1-(4-hydroxy-2-methylphenyl)-4-quinolone-3-carboxylic acid

(500 mg) with morpholine (240 mg) according to a similar manner to that of Example 6.

mp : >300°C

IR (Nujol) : 1705, 1630 $cm^{-1}$

Example 12

5,6,8-Trifluoro-1-(4-hydroxy-2-methylphenyl)-7-piperidino-4-quinolone-3-carboxylic acid (290 mg) was obtained by reacting 5,6,7,8-tetrafluoro-1-(4-hydroxy-2-methylphenyl)-4-quinolone-3-carboxylic acid (500 mg) with piperidine (232 mg) according to a similar manner to that of Example 6.

mp : >300°C

IR (Nujol) : 1705, 1630 $cm^{-1}$

Example 13

5,6,8-Trifluoro-1-(4-hydroxy-2-methylphenyl)-7-(1-pyrrolidinyl)-4-quinolone-3-carboxylic acid (640 mg) was obtained by reacting 5,6,7,8-tetrafluoro-1-(4-hydroxy-2-methylphenyl)-4-quinolone-3-carboxylic acid (500 mg) with pyrrolidine (193 mg) according to a similar manner to that of Example 6.

mp : >300°C

IR (Nujol) : 1725, 1630 $cm^{-1}$

Example 14

To a solution of 5,6,7,8-tetrafluoro-1-(4-hydroxy-2-methylphenyl)-4-quinolone-3-carboxylic acid (200 mg) in dimethylsulfoxide (3 ml) were added dimethylamine hydrochloride (180 mg) and triethylamine (220 mg) at ambient temperature, and the mixture was stirred at 50°C for an hour. To the reaction mixture was added ice-water (30 ml) and the resultant aqueous solution was neutralized with diluted hydrochloric acid. The precipitated crystals were collected by filtration,

washed with water and then dried to obtain 5,6,8-trifluoro-1-(4-hydroxy-2-methylphenyl)-7-dimethylamino-4-quinolone-3-carboxylic acid (190 mg).

mp : 300 to 310°C

IR (Nujol) : 1635 $cm^{-1}$

What we claim is :

1. A compound of the formula :

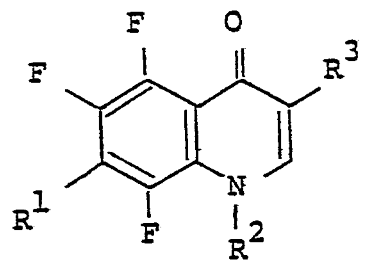

(I)

wherein $R^1$ is di(lower)alkylamino, piperazinyl, piperazinyl having lower alkyl, morpholinyl, pyrrolidinyl or piperidyl,
$R^2$ is cyclo(lower)alkyl or phenyl having lower alkyl and hydroxy, and
$R^3$ is carboxy or protected carboxy,
and physiologically acceptable salts thereof.

2. A compound of claim 1 , which is 1-cyclopropyl-5,6,8-trifluoro-7-(1-piperazinyl)-4-quinolone-3-carboxylic acid or physiologically acceptable salts thereof.

3. A process for the preparation of a compound of the formula :

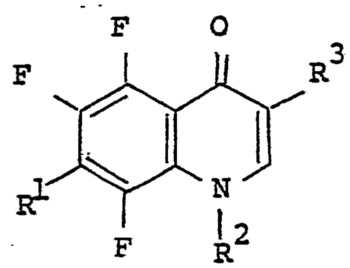

(I)

wherein $R^1$ is di(lower)alkylamino, piperazinyl, piperazinyl having lower alkyl, morpholinyl, pyrrolidinyl or piperidyl,
$R^2$ is cyclo(lower)alkyl or phenyl having lower alkyl and hydroxy, and
$R^3$ is carboxy or protected carboxy,
or salts thereof, which comprises

(1) subjecting a compound of the formula :

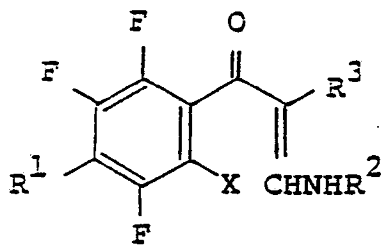

(II)

wherein $R^1$, $R^2$ and $R^3$ are each as defined above,
and
X is halogen,
or salts thereof to cyclization reaction to give a compound of the formula :

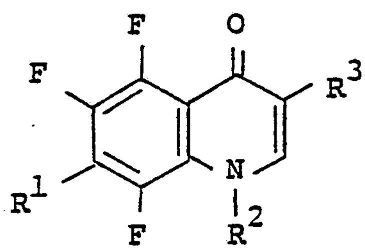

(I)

wherein $R^1$, $R^2$ and $R^3$ are each as defined above, or salts thereof; and

(2) removing a carboxy-protective group from a compound of the formula :

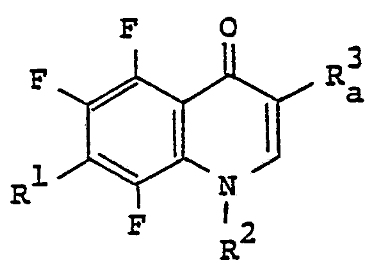

(I-a)

wherein $R^1$ and $R^2$ are each as defined above,
and
$R_a^3$ is protected carboxy,
or salts thereof to give a compound of the formula :

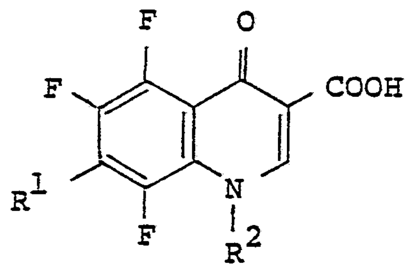

(I-b)

wherein $R^1$ and $R^2$ are each as defined above, or salts thereof; and

(3) reacting a compound of the formula :

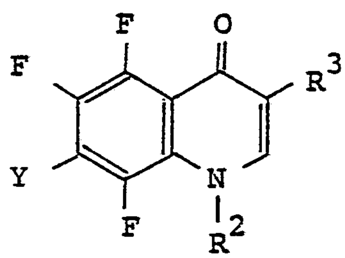

(III)

wherein $R^2$ and $R^3$ are each as defined above,

and

Y is halogen,

or salts thereof with a compound of the formula :

$$R^1_a - H \qquad \text{(IV)}$$

wherein $R^1_a$ is di(lower)alkylamino, 1-piperazinyl, 1-piperazinyl having lower alkyl, morpholino, 1-pyrrolidinyl or piperidino,

or salts thereof to give a compound of the formula :

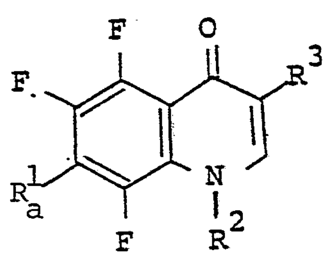

(I-c)

wherein $R^1_a$, $R^2$ and $R^3$ are each as defined above, or salts thereof.

4. An antimicrobial composition comprising a compound of claim 1.

5. A compound of claim 1 for use as antimicrobial agent for human being or animals.

6. Pharmaceutical compositions for the treatment of infectious diseases caused by pathogenic microorganisms comprising a compound of claims 1 or 2.

7. Use of a compound of claims 1 or 2 for the manufacture of a medicament.

European Patent Office

# EUROPEAN SEARCH REPORT

0 247 464

EP 87 10 7108

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 126 355 (BAYER AG)<br>* claims 1-6, 8-10 *<br>--- | 1-7 | C 07 D 215/56<br>A 61 K 31/47 //<br>C 07 C 69/738<br>C 07 C 119/06 |
| Y | EP-A-0 159 174 (WARNER-LAMBERT CO.)<br>* claim 1, in particular page 57, line 11; claims 9, 10 *<br>--- | 1-7 | |
| A | EP-A-0 153 828 (WARNER-LAMBERT CO.)<br>* page 2, line 10 - page 4, line 9 *<br>--- | 1,3-7 | |
| A | EP-A-0 154 780 (ABBOTT LABORATORIES)<br>* claims 1, 20, 21 *<br>--- | 1,3-7 | TECHNICAL FIELDS SEARCHED (Int. Cl.4) |
| P,A | EP-A-0 202 763 (WARNER-LAMBERT CO.)<br>* pages 2-6 *<br>--- | 1,3-7 | C 07 D 215/00<br>A 61 K 31/00 |
| P,X | CHEMICAL ABSTRACTS, vol. 106, no. 25, 22nd June 1987, page 646, column 2, abstract no. 213777c, Columbus, Ohio, US; & JP - A - 62 469 (DAINIPPON PHARMACEUTICAL CO. LTD.) 06-01-1987<br>----- | 1,3-7 | |

The present search report has been drawn up for all claims

| Place of search | Date of completion of the search | Examiner |
|---|---|---|
| BERLIN | 21-08-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document